# EUROPÄISCHE PATENTSCHRIFT

(11) **EP 1 100 958 B1**
(45) Veröffentlichungstag und Bekanntmachung des Hinweises auf die Patenterteilung: **04.01.2006**
(21) Anmeldenummer: 00940297.5
(22) Anmeldetag: 31.05.2000
(51) Int. Cl.: C12Q 1/68, C12Q 1/70, G01N 33/68

(54) **NUCLEINSAUREMOLEKÜLE MIT SPEZIFISCHER ERKENNUNG VON NATIVEM PRPSC, HERSTELLUNG UND VERWENDUNG**
NUCLEIC ACID MOLECULES WITH SPECIFIC IDENTIFICATION OF NATIVE PRPSC , THEIR PRODUCTION AND THE USE THEREOF
MOLECULES D'ACIDES NUCLEIQUES A DETECTION SPECIFIQUE DE LA PRPSC NATIVE, LEUR PRODUCTION ET LEUR UTILISATION

(30) Priorität: 01.06.1999 DE 19925073
(43) Veröffentlichungstag der Anmeldung: 23.05.2001
(73) Patentinhaber: Lasmézas, Corinne Ida, 75019 Paris (FR); Weiss, Stefan, 80796 München (DE)
(72) Erfinder: WEISS, Stefan, D-80796 München (DE)
(74) Vertreter: Henkel, Feiler & Hänzel
(86) Internationale Anmeldenummer: PCT/EP2000/005020
(87) Internationale Veröffentlichungsnummer: WO 2000/073501

(56) Entgegenhaltungen:
- WO-A-97/10505
- WO-A-97/15685
- WO-A-98/35236
- WO-A-98/53838
- WEISS STEFAN ET AL: "RNA aptamers specifically interact with the prion protein PrP." JOURNAL OF VIROLOGY, Bd. 71, Nr. 11, 1997, Seiten 8790-8797, XP002152780 ISSN: 0022-538X in der Anmeldung erwähnt
- KORTH C ET AL: "PRION (PRPSC)-SPECIFIC EPITOPE DEFINED BY A MONOCLONAL ANTIBODY" NATURE,GB,MACMILLAN JOURNALS LTD. LONDON, Bd. 390, Nr. 6655, 6. November 1997 (1997-11-06), Seiten 74-77, XP002069611 ISSN: 0028-0836 in der Anmeldung erwähnt

## Beschreibung

Die vorliegende Erfindung liegt auf dem Gebiet von Nucleinsäuren und deren Derivaten, die mit Prionenproteinen, die am Auftreten der verschiedensten Formen von übertragbarer spongiformer Encephalopathie (TSE) beteiligt sind, wechselwirken können. Insbesondere betrifft die vorliegende Erfindung ein Verfahren zur Selektion und Isolierung von Nucleinsäuremolekülen, die spezifisch zwischen den Isoformen PrP^{c} und PrP^{Sc} unterscheiden können, nach diesem Verfahren erhältliche Nucleinsäuremoleküle sowie pharmazeutische und diagnostische Zusammensetzungen, die diese Nucleinsäuremoleküle enthalten.

Prionen, die vermutliche Ursache von übertragbaren spongiformen Encephalopathien (transmissible spongiform encephalopathies, TSE), wie Scrapie bei Schafen, Mäusen und Hamstern, übertragbare spongiforme Encephalopathie von Rindern (BSE), übertragbare spongiforme Encephalopathie bei Nerzen (TME), Kuru, Gerstmann-Sträußler-Scheinker-Syndrom (GSS), Creutzfeldt-Jakob-Krankheit (CJD) und fatale familiäre Insomnie(FFI) beim Menschen, bestehen hauptsächlich, wenn auch nicht gänzlich aus PrP^{Sc}, einer anomalen Isoform des ubiquitären zellulären Prionenproteins PrP^{c} (zur Übersicht: Lasmézas u. Weiss, 1999; Rieger et al., 1999; Prusiner et al., 1998; Weissmann u. Aguzzi, 1997).

Aus der WO 97/15685 und den dort zitierten Literaturstellen ist bekannt, daß von den Isoformen PrP^{c} und PrP^{Sc} nur PrP^{Sc} zu der gegen Proteinase K beständigen Isoform PrP27-30 verarbeitet werden kann. PrP^{Sc} unterscheidet sich von PrP^{c} bezüglich der Sekundärstruktur, d.h. dem Anteil an α-Helix- und β-Faltblatt-Strukturen. Ferner ist bekannt, daß im Falle des syrisches Goldhamsters PrP27-30 gegenüber PrP^{c} und PrP^{Sc} um 67 Aminosäuren am Aminoterminus verkürzt ist.

Diese Eigenschaft wurde in der WO 97/ 15685 zur Diagnose von mit Prionenproteinen in Verbindung stehenden Erkrankungen verwendet. In einem Test zur Identifizierung und Selektion von spezifischen Nucleinsäuresequenzen, die zur Unterscheidung von zwischen den zellulären Isoformen PrP^{c} und PrP^{Sc} fähig sein sollen, wurde gemäß der Lehre der WO 97 / 15685 an GST fusioniertes PrP^{c} eingesetzt. PrP^{c} und rekombinantes (rec) PrP27-30, bei dem es sich - wie oben erwähnt - um ein lediglich um 67 Aminosäuren am Aminoterminus verkürztes PrP^{c} vom syrischen Goldhamster handelte, wurden als Glutathion-S-Transferase (GST)-Fusionen eingesetzt, um die Bindungseigenschaften der selektierten Nucleinsäuremoleküle zu überprüfen.

Das in der WO 97/15685 beschriebene Verfahren zur Identifizierung und Isolierung von spezifischen Nucleinsäuremolekülen umfaßte hierbei die folgenden Stufen:
- Inkubieren von an Glutathion-S-transferase (GST) fusioniertes PrP^{c} mit einem Pool von Nucleinsäuremolekülen unterschiedlicher Sequenzen,
- Selektieren und Isolieren der zu einer Bindung an GST fusioniertes PrP^{c} fähigen Nucleinsäuremoleküle,
- gegebenenfalls Amplifikation der isolierten Nucleinsäuremoleküle, wobei gegebenenfalls die Inkubationsstufe und die Selektionsstufe wiederholt werden, und
- Bestimmen der Bindungsspezifität der isolierten Nucleinsäuremoleküle für die Isoformen PrP^{c} und PrP^{Sc} oder PrP27-30 oder die Fragmente oder Derivate von diesen.

Die Tatsache, daß die in der Selektion gefundenen Nucleinsäuremoleküle nicht an recPrP27-30 binden, demonstriert jedoch lediglich die Bindung des Nucleinsäuremoleküls an den aminoterminalen Bereich des PrP^{c}-Moleküls.

Vielmehr hat sich gezeigt, daß spezifisch nur an natives PrP^{Sc} bindende Nucleinsäuremoleküle gemäß dem in der WO 97/15685 beschriebenen Verfahren nicht erhalten werden konnten. Ferner kann ein in der WO 97/15685 nachgewiesenes, PrP^{c} spezifisches Nucleinsäuremolekül zur direkten Diagnose von übertragbaren spongiformen Encephalopathien (transmissible spongiform encephalopathies, TSE) nicht eingesetzt werden.

Aufgabe der vorliegenden Erfindung ist daher die Bereitstellung von Nucleinsäuremolekülen, die spezifisch an PrP^{Sc} in nativem Zustand binden und zu dessen Unterscheidung von PrP^{c} fähig sind.

Beschrieben wird ein
Verfahren zur Selektion und Isolierung von Nucleinsäuremolekülen, die zur spezifischen Wechselwirkung mit nativem PrP^{Sc} und zur Unterscheidung von nativem PrP^{c} und nativem PrP^{Sc} fähig sind, wobei das Verfahren die folgenden Stufen umfaßt:
- Inkubieren eines Pools von unterschiedliche Sequenzen aufweisenden Nucleinsäuren mit gereinigten PrP^{Sc}-Präparationen (PPPs),
- Selektieren und Isolieren der entstandenen Protein/Nucleinsäurekomplexe, und
- gegebenenfalls Amplifikation der isolierten spezifischen Nucleinsäuren sowie gegebenenfalls Wiederholen der Inkubations-, Isolierungs- und Amplifikationsstufe.

Vorzugsweise umfaßt das obige Verfahren zusätzlich ein Identifizieren der erhaltenen Nucleinsäuremoleküle in an sich bekannter Weise.

Unter dem Ausdruck "Pool von Nucleinsäuren" verstehen wir ein beliebiges Gemisch aus Nucleinsäuremolekülen unterschiedlicher Sequenzen, vorzugsweise ein Pool von stark entarteten Nucleinsäuren hoher Komplexität. In besonders bevorzugter Weise handelt es sich bei diesem Pool um den auf dem einschlägigen Fachgebiet bekannten RNA Pool M 111.1 (Famulok, 1994). In einer weiteren bevorzugten Ausführungsform handelt es sich bei diesem Pool um einen randomisierten DNA/RNA-Pool, wobei die DNA/RNA-Moleküle so modifiziert sind, daß sie eine erhöhte Stabilität besitzen.

Unter dem Ausdruck "gereinigte PrP^{Sc}-Präparationen (purified PrP^{Sc} preparations; PPPs)" verstehen wir PrP^{Sc}-Präparationen, die durch ein spezielles Aufbereitungsverfahren ohne Verwendung von Proteinase K (PK) gemäß der im folgenden angegebenen Beschreibung gewonnen wurden.

Dabei wird PrP^{Sc} aus Geweben von an einer übertragbaren spongiformen Encephalopathie im klinischen Stadium leidenden Tieren gemäß dem im experimentellen Teil der Beschreibung am Beispiel von TSE und Hirnhomogenat beschriebenen Verfahren in Form eines Pellets isoliert.

Als Nucleinsäuremoleküle können einzel- oder doppelsträngige Nucleinsäuremoleküle, wie RNA, modifizierte RNA, einzelsträngige DNA oder doppelsträngige DNA verwendet werden.

Der Ausdruck "natives PrP^{c}" umfaßt erfindungsgemäß die ubiquitäre zelluläre Isoform des Prionenproteins sowie Fragmente und Derivate hiervon ungeachtet des Organismus, aus dem sie stammen.

Der Ausdruck "natives PrP^{Sc}" umfaßt die mit übertragbaren spongiformen Encephalopathien in Verbindung stehende anomale Isoform des Prionenproteins sowie natürlich vorkommende Derivate hiervon, ungeachtet des Organismus, aus dem sie stammen.

Unter dem Ausdruck "nativ" verstehen wir in seiner breitesten Form nicht unter denaturierenden Bedingungen vorliegend, wobei denaturierende Bedingungen als die Konformation des Biomoleküls zerstörende Bedingungen anzusehen sind.

Der Ausdruck "Derivate" umfaßt in seiner allgemeinsten Form chemisch modifizierte Versionen der Isoformen der Prionenproteine PrP^{c} und PrP^{Sc} sowie Mutanten dieser Proteine, d.h. Proteine, die sich von den natürlich vorkommenden Prionenprotein-Isoformen in einer oder mehreren Positionen der Aminosäuresequenz unterscheiden, sowie Proteine, die Deletionen und/oder Insertionen gegenüber den natürlich vorkommenden Prionenprotein-Isoformen besitzen. Derartige Mutanten können durch rekombinante DNA-Technologie hergestellt worden sein oder es kann sich um natürlich vorkommende Mutanten handeln. Der Ausdruck Derivate umfaßt auch Proteine, die modifizierte Aminosäuren enthalten, oder Proteine, die durch Glykosylierung, Phosphorylierung oder dergleichen modifiziert sind.

Die als Sonde bzw. Target zur Isolierung von Nucleinsäuremolekülen, die zur spezifischen Wechselwirkung mit nativem PrP^{Sc} und zur Unterscheidung von nativem PrP^{c} und nativem PrP^{Sc} fähig sind, verwendeten gereinigen PrP^{Sc}-Präparationen (PPPs), die als infektiöse Fraktion hauptsächlich eine aggregierte Form von PrP^{Sc} darstellen, können beispielsweise aus mit Scrapie infizierten Hamsterhirnen ohne Behandlung mit Proteinase K in Form eines Pellets isoliert werden.

Nach dem Verfahren lassen sich Nucleinsäuremoleküle isolieren, die zu unterscheiden vermögen zwischen einem nativen ubiquitären PrP^{c} und der nativen anomalen Isoform PrP^{Sc}, die mit den oben genannten übertragbaren spongiformen Encephalopathien assoziiert sind, wie Scrapie bei Schafen, Mäusen und Hamstern, bovine spongiforme Encephalopathie bei Rindern (BSE), übertragbare "Mink (Nerz)"-Encephalopathie bei Nerzen (TME), Kuru, Gerstmann-Sträußler-Scheinker-Syndrom (GSS), Creutzfeldt-Jakob-Krankheit (CJD) inklusiv der neuen Variante (nvCJD) und fatale familiäre Insomnie(FFI) beim Menschen,chronischer Kräfteverfall (CWD) beim Maultier, Hirsch, und Elch und übertragbare spongiforme Encephalopathie bei Katzen (FSE).

Das Inkubieren des Nucleinsäurepools mit den PPPs kann auf verschiedene Weise erfolgen.

In einer bevorzugten Ausführungsform werden die in Pelletform vorliegenden PPPs in einem geeigneten Suspendiermittel, beispielsweise Bindungspuffer, aufgenommen und mit dem Nucleinsäurepool unter Bildung einer kolloidalen Lösung inkubiert.

Die PPPs können jedoch auch auf Matrix, beispielsweise einem Gel oder einem Harz, immobilisiert werden. Die Immobilisierung erfolgt dabei in dem Fachmann auf dem einschlägigen Fachgebiet an sich bekannter Weise. Beispielsweise können die PPPs kovalent an die Matrix oder durch eine spezifische Wechselwirkung zwischen einer auf der Matrix vorhandenen Gruppe und einer Domäne in den Proteinen der PPPs, die die Gruppe der Matrix spezifisch erkennt, an die Matrix gebunden werden. Eine derartige Domäne kann in dem Fachmann auf dem einschlägigen Fachgebiet bekannter Weise beispielsweise im Rahmen rekombinanter DNA-Techniken an die Precursormoleküle von PPP anfusioniert werden.

Im Rahmen einer bevorzugten Ausführungsform des Verfahrens kann eine Vorselektion zur Entfernung von nichtspezifisch bindenden Nucleinsäuremolekülen durchgeführt werden. Dies kann beispielsweise mit "Pseudo-PPPs" geschehen, die aus nicht mit Scrapie infizierten Hirnhomogènaten gewonnen wurden. Diese Vorselektion kann, wie oben für die Inkubation mit den PPPs beschrieben, mit "Pseudo-PPPs" in kolloidaler Lösung oder in an Matrix immobilisierter Form erfolgen.

Die entstandenen Protein/Nucleinsäurekomplexe können in auf dem einschlägigen Fachgebiet bekannter Weise isoliert werden. Beispielsweise können im Falle von immobilisierten PPPs nicht gebundene Nucleinsäuremoleküle nach der Inkubation mit einem geeigneten Puffer ausgewaschen werden. Danach können die gebundenen Nucleinsäuremoleküle beispielsweise mit 8M Harnstoff eluiert und anschließend durch Phenolextraktion und Fällung weiter gereinigt werden. Im Falle einer Inkubation mit PPPs in kolloidaler Lösung können die gebildeten Komplexe durch Ultrazentrifugation im Pellet gewonnen werden, wovon die Nucleinsäuremoleküle extrahiert werden können. Alternativ können die gebildeten Komplexe nach Inkubation der Nucleinsäuremoleküle mit den PPPs filtriert werden.

Ferner können die erhaltenen Nucleinsäuremoleküle gegebenenfalls beispielsweise durch in vitro-Transcription, reverse Transcription oder PCR oder eine Kombination dieser Techniken amplifiziert und anschließend die erhaltenen Produkte ein weiteres Mal den Schritten Inkubation und Isolierung (= Selektion) unterzogen werden. Durch eine derartige Wiederholung der Schritte Inkubation, Isolierung und Amplifikation können die spezifisch bindendenen Nucleinsäuremoleküle angereichert werden.

So kann es beispielsweise durch Amplifikation in einem ersten Zyklus zu einer Anreicherung von < 0,01 %, im zweiten von 5 % und im dritten von >30% Nucleinsäureprodukten im gegen PPPs gerichteten RNA-Pool kommen (d.h. im ersten Zyklus binden < 0,01 %, im zweiten Zyklus binden 5 % und im dritten Zyklus binden > 30 % der Nucleinsäuremoleküle des RNA-Pools spezifisch an die PPPs).

Die durch Selektion und Isolierung erhaltenen-Nucleinsäuremoleküle werden in einer dem Fachmann an sich bekannten Weise identifiziert.

Gegenstand eines ersten Aspekts der vorliegenden Erfindung sind Nucleinsäuremoleküle der folgenden Formel: 
5' GGAGCUCAGCCUUCACUGCGGCAAAGGCGGGAAAGCGUGCUAACGUGG
AAAGCUACUCCCACGUUGUACGCGUCGCAGAUCAUUGAGUGAGGGGCACC
ACGGUCGGAUCCUC 3'
 Gegenstand eines weiteren Aspekts der vorliegenden Erfindung sind Nucleinsäuremoleküle der folgenden Formel:

5' GGCAAAGGCGGGAAAGCGUGCUAACGUGGAAAGCUACUCCCACGUUGU
ACGCGUCGCAGAUCAUUGAGUGAGG 3'

Unter den Umfang der vorliegenden Erfindung fallen hierbei insbesondere die folgenden Manipulationen:
A. Zur Erhöhung der in vitro/in vivo Stabilität der erfindungsgemäßen Nucleinsäuremoleküle und/oder ihrer Beständigkeit gegenüber Nucleasen:
   - vollständiger oder teilweiser Ersatz des Phosphatrückgrats durch ein Phosphorothioatrückgrat;
   - vollständiger oder teilweiser Ersatz der Nucleotidschleifen durch Nichtnucleotidabstandsgruppen (beispielsweise Hexaethylenglycol);
   - vollständiger oder teilweiser Ersatz von Nucleotiden durch 2'-Aminopropyl- oder 2'-Amino- oder 2'-O-Alkyl- oder 2'-Fluororibonucleotide;
   - Substituieren der Pyrimidinbase in 5-Stellung durch 1-Pentenylreste;
   - Überkappen mit modifizierten Nucleotiden (beispielsweise Phosphorothioaten oder invertierten 3'-3'-Nucleotiden);
   - Stabilisieren mit Hilfe von vernetzenden Disulfidbindungen;
   - Mitverwendung chiraler Counterparts;
   - Addieren von Lipidgruppen und Inkorporieren des mit Lipidgruppen derivatisierten Aptamers in Liposomen;
   - Kuppeln an Biopolymerträger, wie beispielsweise Polyethylenglycol;
   - Einkapselung der Nucleinsäuremoleküle.
B. Zur Verbesserung der Affinität und/oder Aktivität der Nucleinsäuremoleküle:
   - Modifizieren der RNA zur Herstellung eines durch Einwirkung von Strahlung vernetzbaren Aptamers beispielsweise durch Einbau von 5-Ioduridin-triphosphat;
   - Kuppeln von RNA an einen kleinen organischen Liganden, der eine weitere Region des Targetmoleküls erkennt und an der Reaktion selbst teilnimmt.
C. Zur Verbesserung der Nachweisbarkeit in diagnostischen Tests:
   - Markieren mit einem Fluorophor;
   - Markieren mit Digoxigenin;
   - Markieren mit Biotin.

Bei dem oben als erfindungsgemäß bevorzugt beschriebenen Nucleinsäuremolekül der oben angegebenen Formel handelt es sich um ein Nucleinsäuremolekül, das eine Stamm-Schleifen-Struktur bilden kann und das spezifisch mit nativem, nicht mit Proteinase K behandelten PrP^{Sc}, jedoch nicht mit PrP27-30, der nach Proteinase K-Verdau entstehenden beständigen Form von PrP^{Sc}, und nativem authentischem PrP^{c} reagiert. Dabei können die Prion-Präparationen beispielsweise vom Hamster, Maus, Rind, oder Menschen stammen. Dieses spezifisch an natives PrP^{Sc} bindende Nucleinsäuremolekül wird in der Literatur (vgl. beispielsweise WO 97/ 15685 und die darin angegebenen Referenzen sowie Tuerk und Gold, 1990) häufig auch als RNA-Aptamer bezeichnet.

Das erfindungsgemäße Aptamer reagiert jedoch beispielsweise mit denaturiertem PrP^{c} und PrP^{Sc} von Hamstern, Rindern (PrP^{BSE}), Menschen (PrP^{CJD}) und Mäusen, was die spezifische Erkennung von nur nativem PrP^{Sc} belegt.

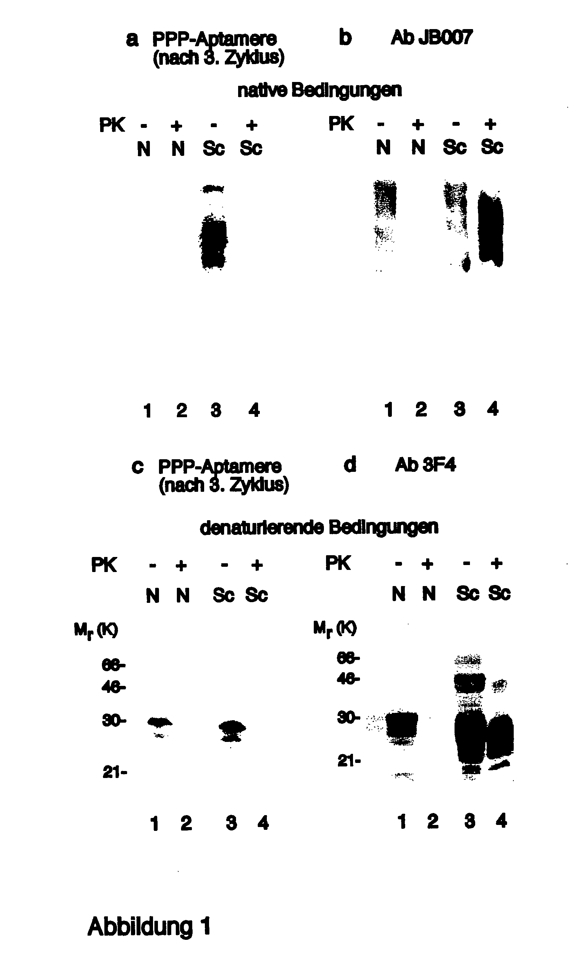

Der Nachweis, daß die erfindungsgemäßen RNA-Aptamere spezifisch an die Prionenproteine PrP^{Sc}, jedoch nicht an PrP^{c} und das nach Proteinase K-Verdau entstehende beständige PrP27-30 banden, wurde beispielsweise durch North-Western-Blot-Analyse mit Hirnhomogenaten von nicht infizierten Hamstern und an Scrapie im Endstadium leidenden Hamstern mit und ohne Proteinase K durchgeführt. Unter nativen Bedingungen banden beispielsweise nach dem dritten Zyklus selektierte RNA-Aptamere spezifisch an PrP^{Sc} (Abb. 1a), jedoch nicht an PrP^{c} und PrP27-30.

Im Gegensatz hierzu erkannte der polyklonale Antikörper JB007 PrP^{c}, PrP^{Sc} und PrP27-30 (Abb. 1b), was die spezifische Bindung der RNA- Aptamere an PrP^{Sc} belegt. Unter denaturierenden Bedingungen erkannte das erfindungsgemäße Aptamer PrP^{c} und PrP^{Sc} (Abb. 1c), reagierte jedoch nicht mit PrP27-30. Der monoklonale Antikörper 3F4 erkannte PrP^{c}, PrP^{Sc} und PrP27-30 (Abb. 1d).

Beschrieben wird ferner eine Antisense-RNA eines nach dem erfindungsgemäßen Verfahren erhältlichen Nucleinsäuremoleküls. Der Begriff "Antisense-RNA" besitzt dabei die auf dem einschlägigen Fachgebiet allgemein bekannte Bedeutung (vgl. Kumar und Carmichael, 1998, Gerhart et al. 1998 sowie Vanhee-Brossollet und Vaquer, 1998).

Gegenstand eines weiteren Aspekts der vorliegenden Erfindung ist die Verwendung der erfindungsgemäßen Nucleinsäuremoleküle in einem Verfahren zur Untersuchung der Fähigkeit von Makromolekülen, wie Nucleinsäuremolekülen oder Antikörpern, zur selektiven Bindung an PrP^{Sc} unter nativen Bedingungen, wobei das Verfahren die folgenden Stufen umfaßt:
- Ultraschallbehandlung der Gewebeprobe, um eine klare Lösung der Gewebeprobe herzustellen,
- Trennung der klaren Lösung durch native Polyacrylamidelektrophorese (vorzugsweise unter Verwendung eines PAA-Gradienten (gewöhnlich 3,5 bis 12% PAA, vorzugsweise 4 bis 12% PAA und insbesondere 6 bis 12% PAA)), um einzelne Proteinbanden zu erhalten, und nachgeschaltetes Elektroblotten und
- anschließendes Nachweisen von nativem PrP^{Sc}, vorzugsweise entweder mittels North-Western-Blotting durch Nachweisen von selektiv an natives PrP^{Sc} bindenden Nucleinsäuren oder mittels Western-Blotting durch Nachweisen von nativem PrP^{Sc} mittels eines Antikörpers (z.B. JB 007, SAF 32, 15 B3).
- Alternativ kann die klare Lösung durch native Agarosegelelektrophorese (vorzugsweise unter Verwendung eines Agarosegradienten) getrennt werden.

Gegenstand eines weiteren Aspekts der vorliegenden Erfindung ist die Verwendung der erfindungsgemäßen Nucleinsäuremoleküle gemäß der obigen Definition als Mittel zur Diagnose von übertragbaren spongiformen Encephalopathien (TSE).

Wie erfindungsgemäß beispielsweise durch North-Western-Blotting oder Dot-Blot-Technik festgestellt werden kann, können die erfindungsgemäßen Nucleinsäuremoleküle selektiv beispielsweise natives PrP^{Sc/CJD/BSE} von Hamster, Maus, Mensch und Rind erkennen.

Im Gegensatz dazu konnten das recPrP-I-Aptamer (entspricht RNA-Aptamer Motiv I (Ap 1) bei Weiss et al., 1997) und PrP-Antikörper sowohl natives PrP^{c} als auch PrP^{Sc} erkennen. Unter denaturierenden Bedingungen reagieren jedoch die erfindungsgemäßen Nucleinsäuremoleküle und die recPrP-I-Aptamere sowohl mit PrP^{c} als auch PrP^{Sc/CJD/BSE} von Hamster, Maus, Mensch und Rind, was die spezifische Erkennung von PrP^{Sc/CJD/BSE} nur im nativen Zustand belegt.

Gegenstand eines weiteren Aspekts der vorliegenden Erfindung sind ferner diagnostische Zusammensetzungen, die die erfindungsgemäßen Nucleinsäuremoleküle enthalten. Derartige Zusammensetzungen können ferner übliche, auf dem einschlägigen Fachgebiet zur Verwendung in derartigen Zusammensetzungen bekannte Hilfsstoffe enthalten. Die erfindungsgemäßen diagnostischen Zusammensetzungen eignen sich zur Durchführung eines diagnostischen Tests zum Nachweis von Prionenerkrankungen bei unterschiedlichen Tierarten sowie beim Menschen. So können die erfindungsgemäßen diagnostischen Zusammensetzungen in Verfahren zur Diagnose der oben genannten übertragbaren spongiformen Encephalopathien verwendet werden. Ein derartiges Verfahren kann beispielsweise das Inkubieren einer dem Körper der zu untersuchenden Spezies entnommenen Probe mit einem erfindungsgemäßen Nucleinsäuremolekül und ein anschließendes Bestimmen der Wechselwirkung der Nucleinsäuremoleküle mit nativem PrP^{Sc} umfassen. Hierbei können die erfindungsgemäßen Nucleinsäuremoleküle im Rahmen eines spezifischen und einfachen Verfahrens ohne die Notwendigkeit einer vorgeschalteten Verdauung mit Proteinase K an nativem Material eingesetzt werden. Die Bestimmung der Wechselwirkung der Nucleinsäuremoleküle mit nativem PrP^{Sc} kann dabei beispielsweise durch Messung der ligandengebundenen Radioaktivität, mittels fluorometrischer Bestimmung mithilfe eines Biosensors oder unter Verwendung einer geeignet angepaßten Enyzme-linked-Oligonucleotid-Assay (ELOA) Technik erfolgen. Ferner ist es möglich, erfindungsgemäß die Menge an nativem PrP^{Sc} quantitativ durch Messung der Radioaktivität, Fluoreszenzanisotropie oder durch densitometrische Messung mit Hilfe eines ELOA-Tests in einer Probe zu bestimmen.

In einer bevorzugten Ausführungsform kann die zu untersuchende Probe aus den verschiedensten Organen und Geweben beispielsweise in Formalin fixierter oder in gefrorener Form stammen, beispielsweise aus Gehirn, Mandeln, Darm, Dura mater, Lymphknoten, Nerven, Milz, Placenta, Magen, Augen, Rückenmark oder Peyer'schen Plaques. Ferner kann es sich bei den Proben um Körperflüssigkeiten, vorzugsweise Blut, Cerebrospinalflüssigkeit, Milch oder Sperma, handeln.

Sofern Hirn als Probe verwendet werden soll, wird die Diagnose in der Regel post mortem durchgeführt. Es ist jedoch auch möglich, die diagnostische Untersuchung in From von Hirnbiopsien am lebenden Organismus durchzuführen. Untersuchungen der oben genannten Körperflüssigkeiten sowie von Organproben kann ebenfalls am lebenden Organismus durchgeführt werden.

Gegenstand eines weiteren Aspekts der vorliegenden Erfindung sind pharmazeutische Zusammensetzungen, die die erfindungsgemäßen Nucleinsäuremoleküle enthalten. Derartige Zusammensetzungen können darüber hinaus pharmazeutisch akzeptable Träger und andere auf dem einschlägigen Fachgebiet bekannte Hilfsstoffe enthalten. Als Hilfsstoffe eignen sich insbesondere solche, die die in vivo-Halbwertszeit der die erfindungsgemäßen Nucleinsäuremoleküle erhöhen oder ein spezifisches Binden der erfindungsgemäßen Nucleinsäuremoleküle verbessern. Spezifische Beispiele für solche Hilfsstoffe sind die oben im Zusammenhang mit der Erhöhung der Stabilität und/oder der Veränderung der biochemischen und/oder biophysikalischen Eigenschaften der erfindungsgemäßen Nucleinsäuremoleküle genannten Stoffe. Ferner können die der erfindungsgemäßen Nucleinsäuremoleküle in herkömmliche Materialien eingekapselt oder in Liposomen in dem Fachmann auf dem einschlägigen Fachgebiet bekannter Weise eingearbeitet werden.

Diese erfindungsgemäßen pharmazeutischen Zusammensetzungen eignen sich zur Behandlung der oben beschriebenen übertragbaren spongiformen Encephalopathien. So kann beispielsweise durch orale oder parenterale (beispielsweise intravenöse) Verabreichung der erfindungsgemäßen pharmazeutischen Zusammensetzungen die Produktion von PrP^{Sc} in infizierten Zellen unterdrückt werden.

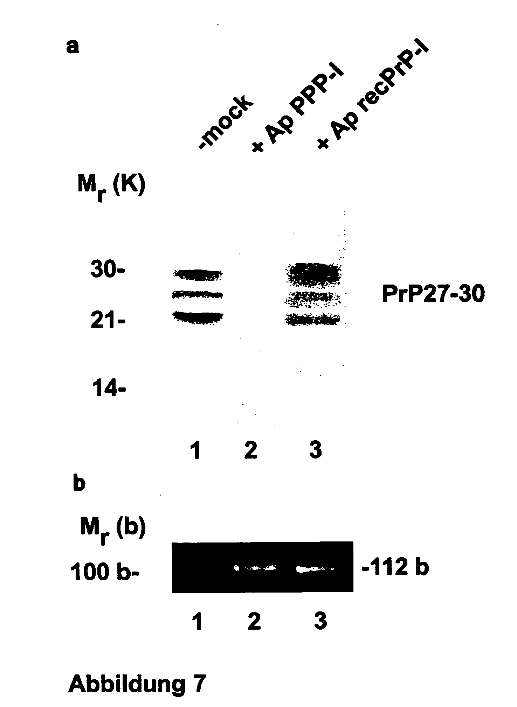

So wurden erfindungsgemäß beispielsweise mit Scrapie infizierte Neuroblastom-Zellen [ScN₂a (MHM-2)] mit den RNA-Transkriptionsplasmiden pCIneo (mock), pCIneo_PPP-I and pCIneo_recPrP-I transfiziert. Dieser transgene Zelltyp enthält ein chimäres Maus-Hamster-Maus-PrP-Konstrukt, so daß mit Hilfe spezifischer Antikörper, wie 3F4, der zwar Hamster-, aber nicht Maus-PrP erkennt, zwischen endogen exprimiertem Maus-PrP und transgen exprimiertem Hamster-PrP unterschieden werden kann. Die Western-Blot-Analyse von Proteinpräparationen dieser Zellen 48 h nach Transfektion ergab nach einer Proteinase K (pK)-Behandlung, daß mit pCIneo_PPP-I, welches das PPP-I-Aptamer (112-mer; SEQ ID Nr. 2) produziert, transfizierte Zellen kein PrP^{Sc} produzierten (Abb. 7a, Spur 2). Im Gegensatz dazu hatte das Aptamer recPrP-I (vgl. RNA-Aptamer Motiv-I (Ap 1) Weiss et al., 1997) keine Wirkung auf die Produktion von PrP^{Sc} (Abb.7a, Spur 3) ebenso wie die Mockkontrolle, bei der mit dem Vektor pCIneo transfiziert wurde (Abb. 7a, Spur 1).

Zur Analyse der Spiegel von PPP-I- und recPrP-I-RNA in den Neuroblastomzellen [(ScN₂a (MHM-2)], die mit pCIneo (Abb. 7b, Mock, Spur 1), pCIneo_PPP-I (Abb. 7b, Spur 2) und pCIneo_recPrP-I (Abb. 7b, Spur 3) transfiziert waren, wurde die gesamte RNA zeitgleich zur Herstellung der Proteinpräparationen gereinigt. Nach reverser Transkription dieser RNA mit dem PCR-Primer I und PCR-Amplifikation der cDNA mit den PCR-Primern I und II zeigte eine Analyse mittels Agarosegelelektrophorese, daß die mit pCIneo_PPP-I und pCIneo_recPrP-I transfizierten Zellen die gleichen Spiegel von PPP-I-RNA (112-mer; SEQ ID Nr. 2) (Abb. 7b, Spur 2) und recPrP-I-RNA (Abb. 7b, Spur 3) bildeten, während die mit pCIneo transfizierten Zellen (Mock) keine Spiegel an PPP-I-RNA und recPrP-I-RNA (Abb. 7b, Spur 1) zeigten.

Diese Daten zeigen, daß die Transkription von PPP-I-RNA in ScN₂a- Zellen den PrP^{Sc}-Spiegel und den PrP^{c}-Spiegel (Daten nicht gezeigt) unter die Nachweisgrenze absenkt. Dieser Effekt ist dabei nicht auf ein höheres Transkriptionsniveau von PPP-I-RNA, verglichen mit recPrP-I-RNA, sondern auf die eine PrP^{Sc}/PrP^{c}-Expression unterbindende Eigenschaft von PPP-I-RNA zurückzuführen.

Diese Daten legen nahe, daß das PPP-I-Aptamer durch spezifische Wechselwirkung mit nativem PrP^{Sc} und vermutlich auch mit frühen PrP-Synthesezwischenprodukten stark in den Bildungsprozess von nativem PrP^{Sc} in vivo eingreift. Das Aptamer kann ferner in den Metabolismus von PrP^{c} eingreifen. Daraus ergibt sich die Verwendbarkeit des erfindungsgemäßen Aptamers als therapeutisches Mittel gegen TSE.

Da das PPI-I-Aptamer nachweislich PrP^{Sc}-Moleküle "abfangen" kann, kann es auch als RNA-Decoy (engl. für Lockvogel, Köder) bzw. PPI-I Decoy bezeichnet werden. DNA Decoys sind in der Literatur beispielsweise bei King et al., 1998, beschrieben.

Die Wechselwirkung der erfindungsgemäßen Nucleinsäuresequenzen unter denaturierenden Bedingungen mit sowohl PrP^{c} als auch PrP^{Sc} sowie die fehlende Wechselwirkung mit PrP27-30, dem je nach Spezies im Vergleich zu PrP^{Sc} 67 bis 76 Aminosäuren am aminoterminalen Ende fehlen, legt nahe, daß die Erkennung an einem Epitop im aminoterminalen Teil von PrP^{Sc} (AS23/25 bis 90-101) erfolgt. Die Erkenntnis, daß vier Arten, Mensch, Rind, Hamster und Maus (Daten werden hier nicht gezeigt) vom Aptamer erkannt werden, begünstigt die Annahme, daß es sich um eine hoch konservierte Erkennungsstelle in PrP unterschiedlicher Säugetiere handelt, die von den erfindungsgemäßen Nucleinsäuremolekülen erkannt wird.

### Beschreibung der Zeichnungen

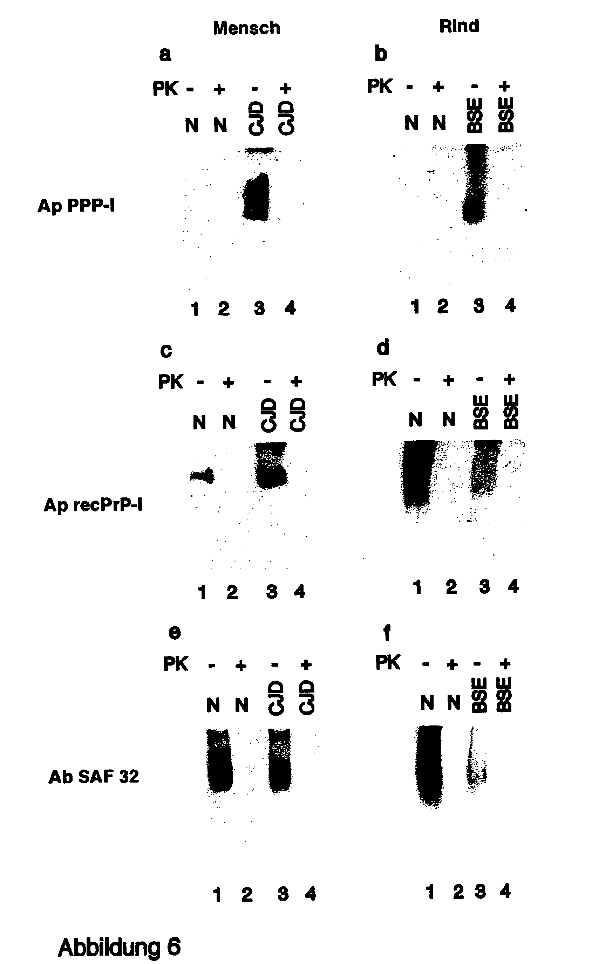

Verzeichnis der Abkürzungen:
1. Ab 3F4 = monoklonaler Antikörper 3F4
2. Ab 15B3 = monoklonaler Antikörper 15B3
3. Ab JB007 = polyklonaler Antikörper JB007
4. Ab SAF32 = monoklonaler Antikörper SAF32
5. Ap Ctrl-I = Kontrollaptamer (112-mer; SEQ ID Nr. 4)
6. Ap PPP-I = Aptamer PPP-I (112-mer; SEQ ID Nr. 2)
7. Ap recPrP-I = RNA-Aptamer Motif-I (Ap1) (vgl. Weiss et al., 1997)
8. BSE = Hirnhomogenat aus einem an BSE-erkrankten Rind
9. CJD = Hirnhomogenat von einem Patienten, der an sporadischer CJD erkrankt ist
10. Sc = Hirnhomogenat von einem an Scrapie erkrankten Hamster
11. Mᵣ(b) = Molekulargewichtsstandard in Basen
12. Mᵣ(K) = Molekulargewichtsstandard in kDa
13. N = normales Hirnhomogenat aus Hamster (Abb. 1, 3, 4, 5) bzw. Mensch und Rind (Abb.6)
14. PK = Proteinase K
15. -pk = ohne Proteinase K-Verdau
16. +pk = mit Proteinase K-Verdau
17. Pool RNA = randomisierter RNA-Pool (Famulok; 1994)
18. PPP = purified PrP^{Sc} preparation (gereinigte PrP^{Sc} Präparation)
19. PrP27-30 = Proteinase-resistentes Prion-Protein

Abb. 1 zeigt die North-Western- und Western-Blot-Analyse von Hirnhomogenaten von nichtinfizierten und an Scrapie leidenden Hamstern mit PPP-Aptameren (nach dem 3. Zyklus) und PrP-spezifischen Antikörpern unter nativen und denaturierenden Bedingungen. Hirnhomogenate von nichtinfizierten Hamstern ohne (Spuren 1) und mit pK (Spuren 2) und Hirnhomogenate von an Scrapie leidenden Hamstern ohne (Spuren 3) und mit pK (Spuren 4) wurden auf nativen PAA-Gradientengelen, 6 - 12 % (a, b) und denaturierenden SDS-12 % PAA-Gelen (c, d) aufgetrennt, geblottet und mit radioaktiv markierten PPP-Aptameren (nach dem 3. Zyklus) (a, c) und den Antikörpern JB007 (b) und 3F4 (d) entwickelt. Die Tatsache, daß der JB007 Antikörper diesselben Proteinbanden erkennt wie die PPP-Aptamere (vgl. Abb. 1a,b, Spuren 3) demonstriert, daß die PPP Aptamere spezifisch natives PrP^{Sc} erkennen können.

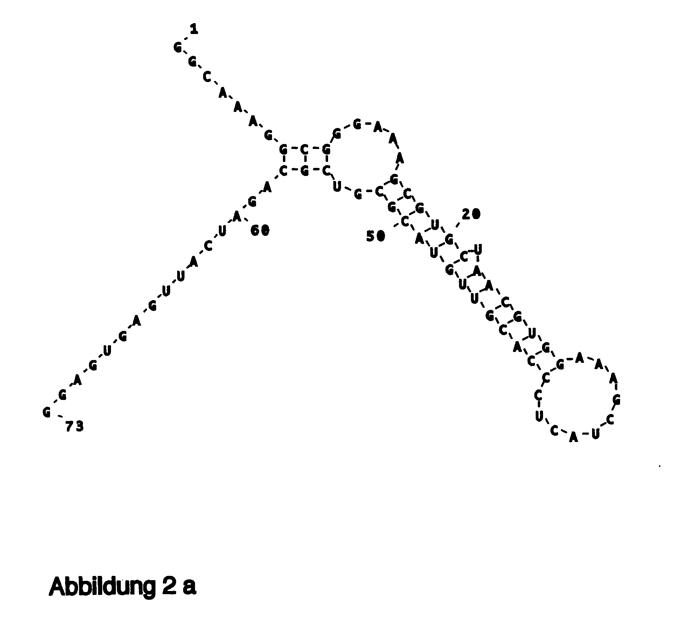

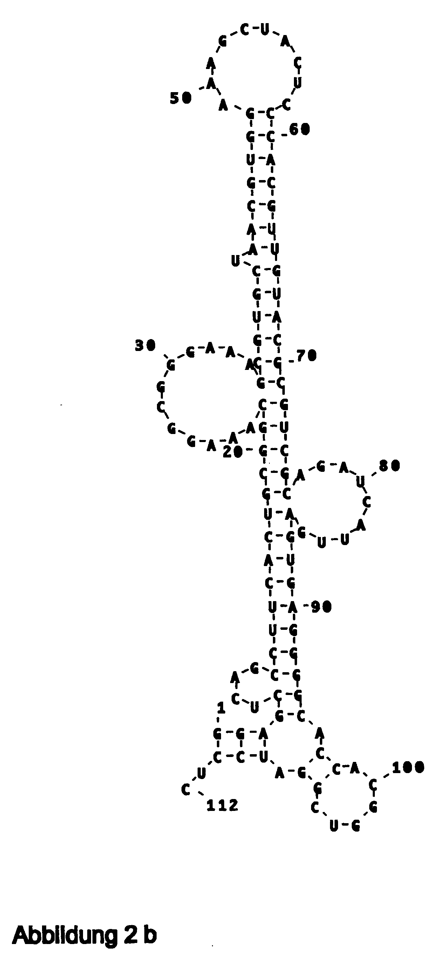

Abb. 2a zeigt ein zweidimensionales Modell einer Stamm-Schleifen-Struktur, das aus dem 73-mer Aptamer PPP-I (SEQ ID Nr. 1) erstellt wurde.

Abb. 2b zeigt ein zweidimensionales Modell einer Stamm-Schleifen-Struktur, das aus dem 112-mer Aptamer PPP-I mit den feststehenden Regionen am 5'- und 3'-Terminus (SEQ ID Nr. 2) erstellt wurde.

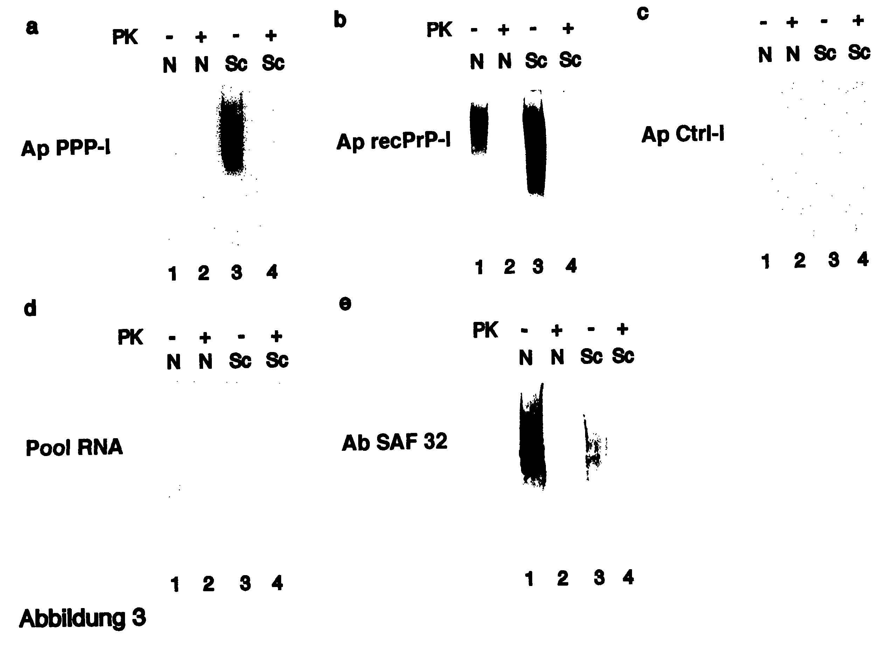

Abb. 3 zeigt die North-Western- und Western-Blot-Analyse von Hirnhomogenaten von nichtinfizierten und an Scrapie leidenden Hamstern mit PPP-I-Aptamer (112-mer, SEQ ID Nr. 2), recPrP-I-Aptamer (vgl. RNA-Aptamer Motiv I (Ap 1) bei Weiss et al., 1997), Kontrollaptamer Ctrl-I (112-mer, SEQ ID Nr. 4), der Pool-RNA und dem Antikörper SAF32 unter nativen Bedingungen. Hirnhomogenate von nichtinfizierten Hamstern ohne (Spuren 1) und mit pK (Spuren 2) und Hirnhomogenate von an Scrapie leidenden Hamstern ohne (Spuren 3) und mit pK (Spuren 4) wurden auf nativen PAA-Gradientengelen, 4 -12 %, aufgetrennt, geblottet und mit radioaktiv markiertem PPP-I-Aptamer (112-mer, SEQ ID Nr. 2) (a), recPrP-I-Aptamer(6)(b), Kontrollaptamer Ctrl-I (112-mer, SEQ ID Nr. 4) (c), der radioaktiv markierten Pool-RNA (d) und dem Antikörper SAF 32 (e) entwickelt. Es zeigt sich, daß lediglich mit dem PPP-I-Aptamer selektiv eine Bindung an natives PrP^{Sc} aus Hirnhomogenaten von an Scrapie leidenden Hamstern ohne pK-Behandlung (a, Spuren 3) festgestellt wird.

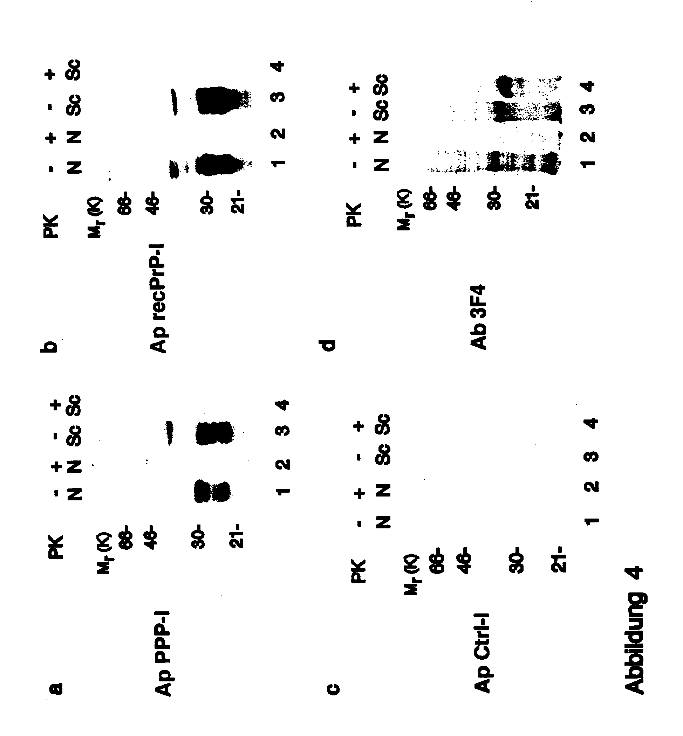

Abb. 4 zeigt die North-Western- und Western-Blot-Analyse von Hirnhomogenaten von nichtinfizierten und an Scrapie leidenden Hamstern mit radioaktiv markierten PPP-I-Aptamer (112-mer, SEQ ID Nr. 2), recPrP-I-Aptamer (vgl. Weiss et al., 1997), Kontrollaptamer Ctrl-I (112-mer, SEQ ID Nr. 4) und dem Antikörper 3F4 unter denaturierenden Bedingungen. Hirnhomogenate von nichtinfizierten Hamstern ohne (Spuren 1) und mit pK (Spuren 2) und Hirnhomogenate von an Scrapie leidenden Hamstern ohne (Spuren 3) und mit pK (Spuren 4) wurden auf SDS-12 %-PAA-Gelen aufgetrennt, geblottet und mit radioaktiv markierten PPP-I-Aptamer (112-mer, SEQ ID Nr. 2) (a), recPrP-I-Aptamer (b), Kontrollaptamer Ctrl-I (112-mer, SEQ ID Nr. 4) (c) und dem Antikörper 3F4 (d) entwickelt. Es zeigt sich, daß unter denaturierenden Bedingungen das PPP-I-Aptamer (112-mer, SEQ ID Nr. 2) als auch das rec PrP-I-Aptamer (112-mer, SEQ ID Nr. 4) vergleichbar gut an PrP^{c} und PrP^{Sc} aus Hirnhomogenaten von gesunden und an Scrapie leidenden Hamstern binden (a,b, Spuren 3).

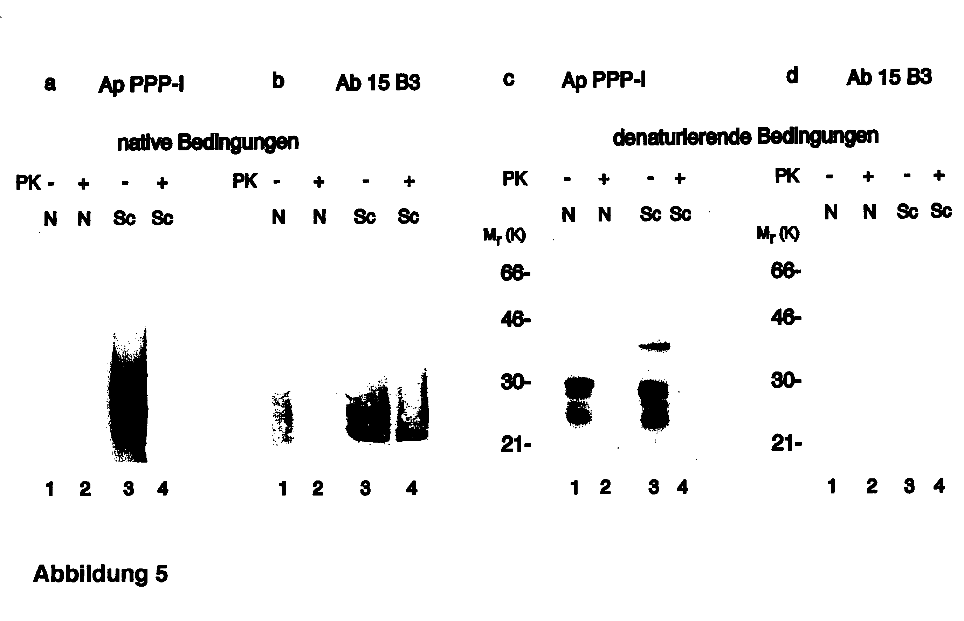

Abb. 5 zeigt einen Vergleich des PPP-I-Aptamers (112-mer; SEQ ID Nr. 2) mit dem Antikörper 15 B3 (Korth et al., 1997) bezüglich der Bindungsfähigkeit an PrP^{c}, PrP^{Sc} und PrP27-30 unter nativen und denaturierenden Bedingungen durch North-Western- und Western-Blot-Analyse. Hirnhomogenate von nichtinfizierten Hamstern ohne (Spuren 1) und mit pK (Spuren 2) und Hirnhomogenate von an Scrapie leidenden Hamstern ohne (Spuren 3) und mit pK (Spuren 4) wurden auf nativen PAA-Gradientengelen, 3,5 - 12 %, (a,b) und SDS-12 % PAA-Gelen (c,d) aufgetrennt und mit dem radioaktiv markierten PPP-I-Aptamer (112-mer, SEQ ID Nr. 2) (a,c) und dem Antikörper 15 B3 (b,d) entwickelt. Es zeigt sich, daß das PPP-I-Aptamer unter nativen Bedingungen selektiv an PrP^{Sc} aus Hirnhomogenaten von an Scrapie leidenden Hamstern (a, Spur 3) bindet, während der 15B3 Antikörper natives PrP^{c} (b, Spur 1), PrP^{Sc} (b, Spur 3) und PrP 27-30 (b, Spur 4) erkennt. Der 15B3 Antikörper erkennt wie beschrieben (Korth et al., 1997) weder PrP^{c}, PrP^{Sc} noch PrP 27-30 unter denaturierenden Bedingungen (d, Spuren 1-4), während des PPP-I-Aptamer unter denaturierenden Bedingungen PrP^{c} (c, Spur 1) und PrP^{Sc} (c, Spur 3) erkennt..

Abb. 6 zeigt die North-Western- und Western-Blot-Analyse von Hirnhomogenaten von Menschen, CJD-Patienten, Kühen und an BSE leidenden Rindern. Hirnhomogenate von Menschen ohne (Spuren 1) und mit pK (Spuren 2) und Hirnhomogenate von CJD-Patienten ohne (Spuren 3) und mit pK (Spuren 4) wurden auf nativen PAA-Gradientengelen, 4 - 12 %, aufgetrennt, geblottet und mit radioaktiv markiertem PPP-I-Aptamer (112-mer, SEQ ID Nr. 2) (a), recPrP-I-Aptamer (c) (Weiss et al., 1997) und dem Antikörper SAF 32 (e) entwickelt. Hirnhomogenate von Kühen ohne (Spuren 1) und mit pK (Spuren 2) und Hirnhomogenate von an BSE leidenden Rindern ohne (Spuren 3) und mit pK (Spuren 4) wurden auf nativen PAA - Gradientengelen, 4 - 12 %, getrennt, geblottet und mit radioaktiv markiertem PPP-I-Aptamer (112-mer, SEQ ID Nr. 2) (b), recPrP-I-Aptamer (d) und dem Antikörper SAF 32 (f) entwickelt. Es zeigt sich, daß das PPP-I-Aptamer selektiv an natives PrP^{CJD/BSE} aus Hirnhomogenaten von CJD-Patienten und an BSE leidenden Rindern (a,b, Spuren 3) bindet.

Abb. 7 zeigt eine Western-Blot- und RT-PCR-Analyse von Protein- und RNA-Präparationen aus mit Scrapie infizierten Neuroblastomzellen [ScN₂a (MHM-2)], die mit Plasmiden, welche die Aptamere PPP-I und recPrP-I kodieren, transfiziert wurden. (a) Die Proteinpräparationen aus mit Scrapie infizierten Neuroblastomzellen, die mit pCIneo (Mock, Spur 1), pCIneo_PPP-I (Spur 2) und pCIneo_recPrP-I (Spur 3) transfiziert waren, wurden nach der Behandlung mit pK auf einem SDS-12 % PAA-Gel analysiert, auf Nitrocellulose geblottet und mit dem für PrP spezifischen Antikörper 3F4 entwickelt. (b) RNA wurde aus [ScN₂a (MHM-2)]-Zellen, die wie unter (a) beschrieben transfiziert waren, extrahiert und einer RT-PCR-Reaktion unter Verwendung der PCR-Primer I und II unterzogen. Die erhaltene DNA wurde auf einem 2,5 % Agarosegel analysiert. Es zeigt sich, daß in Anwesenheit des PPP-I-Aptamers kein PrP^{Sc} mehr synthetisiert wird (a, b, Spuren 2), wogegen die Anwesenheit des rec PrP-I-Aptamers keinen Einfluß auf die Synthese von PrP^{Sc} hat (a, b, Spuren 3).

Die vorliegende Erfindung wird im folgenden unter Bezug auf die Zeichnungen anhand von nicht beschränkend wirkenden Beispielen näher erläutert.

### Beispiele

### Beispiel 1

Herstellung des für PrP^{Sc} spezifischen PPP-I-Aptamers Materialien

### Präparation der PPPs

PPPs wurden beispielsweise aus Hirnen von intracerebral mit Scrapie inokulierten Nagetieren, z.B. Hamstern, im klinischen Endstadium in bekannter Weise (Lasmezas et al., 1997) oder gemäß dem im folgenden beschriebenen Verfahren hergestellt, wobei keine Verdauung mit Proteinase K durchgeführt wurde. Die "Pseudo"-PPPs wurden nach einem analogen Verfahren aus nichtinfizierten Hamsterhirnen erzeugt, wobei jedoch in keinem Fall eine Verdauung mit Proteinase K durchgeführt wurde.

Vor der in-vitro-Selektion wurden die PPPs und "Pseudo"-PPPs einmal mit 300 µl Bindungspuffer (Weiss et al., 1997) bestehend aus 8 mM Na₂HPO₄; 0.87 mM KH₂PO₄; 136 mM NaCl; 112.6 mM KCl; 2 mM DTT; 2 mM MgCl₂ gewaschen.

### Präparation der Hirnhomogenate

Es wurden Hirnhomogenate von nichtinfizierten Hamstern, intracerebral mit dem Scrapiestamm 263K inokulierten Hamstern, nichtinfizierten Mäusen; mit dem Scrapiestamm C506 M3 inokulierten Mäusen, nichtinfizierten Rindern, an BSE leidenden Rindern (von M. Dawson, CVL, GB), einem an einer nicht neurologischen Krankheit gestorbenen Menschen und einem an sporadischer CJD leidenden Patienten präpariert.

Hirnmaterial wurde zur Herstellung einer 20%igen (w/v) Lösung in einer 5%igen isotonischen Glucoselösung homogenisiert. Anschließend wurden 200 µl 20%iges Hirnhomogenat in isotonischer Glucoselösung mit 200 µl 20%iger NaCl und 200 µl einer frisch hergestellten Lösung aus 20% Sarkosyl, 2% SB3-14 und 2mM Tris-Puffer eines pH-Werts von 7,4 versetzt. Nach einstündigem Inkubieren bei 37 °C wurde das Gemisch auf ein Saccharosekissen aus 10% NaCl, 20% Saccharose, 0,1% SB3-14 und 10 mM Tris-Puffer eines pH-Werts von 7,4 aufgegeben und 2 Stunden bei 30.000 g bei Raumtemperatur zentrifugiert. Der Überstand wurde verworfen und das Pellet zur Weiterverwendung beispielsweise in Bindungspuffer resuspendiert.

### RNA-Pool M111.1

Es wurde der RNA-Pool M111.1 (willkürliche Sequenz: 74 Nucleotide; Basenpermutation 3,56 x 10⁴⁴; Poolkomplexität 1,03 x 10¹⁵ (Famulok, 1994)) verwendet, der für das in-vitro-Selektionsverfahren gegen rekombinantes Hamster-PrP^{c} beschrieben wurde (Weiss et al., 1997).

### Antikörper

Der Antikörper 3F4 wurde von Senetek, PLC, erhalten. Der polyklonale Antikörper JB007 wurde in bekannter Weise hergestellt (vgl. Demaimay et al., 1997). Der Antikörper SAF 32 wurde im Service de Neurovirologie (CEA, Fontenay-aux-Roses, France) und Service de Pharmacologie et Immunologie (CEA, Saclay, France) hergestellt. Er erkennt die Region AS 53 - 93 des Prionenproteins. Der monoklonale Antikörper 15 B3 stammte von Bruno Oesch, Zürich und ist bei Korth et al., 1997 beschrieben.

### In-vitro-Selektion gegen PPPs.

183 µg von radioaktiv markierter [5'-γ- ³²P-ATP] RNA 111.1 (Weiss et al., 1997), die über Sephadex G50 Säulen gereinigt wurde, wurde in einem ersten Vorselektionszyklus mit aus 40 mg nichtinfiziertem Hamsterhirn isolierten "Pseudo-PPPs" (in Phosphatbindungspuffer aufgenommen) (Weiss et al., 1997) in Gegenwart von 120 U RNasin (Roche Diagnostics) inkubiert. Nach einstündiger Inkubation bei 37 °C in einem Überkopfschüttler und 10 minütiger Zentrifugation bei 15000 rpm/min wurde der Überstand mit PPPs, die aus 40 mg Hirn von an Scrapie leidenden Hamstern präpariert wurden, inkubiert. Nach den Stufen Ultrazentrifugation (70000 x g, 30 min) und Waschen wurde die RNA durch 8 M Harnstoff von dem PPP-Pellet eluiert und die RNA durch Phenolextraktion und EtOH-Fällung gereinigt. Der Prozentanteil der Bindung von RNA an die PPPs wurde durch Messen der Radioaktivität (dpm) des RNA-Pellets nach der Ethanolfällung geteilt durch die Gesamtmenge (dpm) der RNA vor dem Selektionszyklus bestimmt. 50% der RNA wurden anschließend mit dem folgenden PCR-Primer I einer reversen Transkriptionsreaktion unterworfen und 50% der hierbei erhaltenen cDNA mit dem PCR-Primer I und dem folgenden PCR-Primer II durch PCR amplifiziert.
PCR Primer I: 5' CCGAATTCTAATACGACTCACTATAGGAGCTCAGCCTTCACTGC 3'
PCR Primer II: 5' GTGGATCCGACCGTGGTGCC 3' 50% der amplifizierten cDNA wurden anschließend in Gegenwart von [α-³²P-UTP] unter Verwendung von T7-RNA-Polymerase in vitro transkribiert. Die hierbei erhaltene RNA wurde in bekannter Weise gereinigt (Weiss et al., 1997). Dazu wurde die RNA auf einem 15 %igen denaturierenden PAA-Gel aufgetrennt, die dominanten RNA-Banden aus dem Gel ausgeschnitten und nachfolgend eluiert und gefällt. 40 µg der einzelnen radioaktiv markierten RNA wurden dem zweiten und dritten Zyklus der Selektion und Amplifikation unterzogen. Nach drei Selektionszyklen wurde die RNA auf der cDNA-Ebene über EcoRI und BamHI in pGEM3-Zf(-) kloniert, wobei 25 Klone erhalten wurden. Zwei dieser Klone erhielten die Bezeichnung pGEM3-Zf(-)_PPP-I und pGEM3-Zf(-)_Ctrl-I. Die Sequenz der einzelnen RNAs auf cDNA-Ebene wurde durch Didesoxysequenzierung bestimmt.

Zur Herstellung der RNAs PPP-I (112-mer; SEQ ID Nr. 2) und Ctrl-I (112-mer; SEQ ID Nr. 4) wurden die cDNAs mit den PCR-Primern I und II amplifiziert und die amplifizierte cDNA in vitro mit T7-RNA-Polymerase transkribiert. Die mit [5'-γ-³²P-ATP] radioaktiv markierte RNA wurde in bekannter Weise gereinigt (Weiss et al., 1997).

### Beispiel 2

### Wirkung des PPP-I-Aptamers auf die Erzeugung von PrP in Scrapie infizierten Neuroblastomzellen [ScN₂a (MHM-2)]

Die cDNA mit Codierung für das PPP-I-Aptamer und das recPrP-I-Aptamer (= pGEM3-Zf(-)-Ap 1, Weiss et al., 1997) wurde aus dem Plasmid pGEM3-Zf(-)-PPP-I und pGEM3-Zf(-)-rec PrP-1 durch EcoRI (5') und *Xba*I (3') exzidiert und in pCIneo (Pharmingen) subkloniert. Die richtige Sequenz der hierbei erhaltenen Plasmid-DNAs pCIneo_PPP-I (entspricht pCIneo_ SAF-I (hinterlegt am 29. März 1999, DSM 12753)) und pCIneo_recPrP-I wurde durch Didesoxysequenzierung bestätigt. Mit Scrapie infizierte Neuroblastomzellen (Scott et al., 1992; ScN₂a, die das Konstrukt MHM-2 (eine Chimäre aus Maus-Hamster-Maus enthält); diese [ScN₂a (MHM-2)]-Zellinie wurde von Prof. Dr. S. B. Prusiner zur Verfügung gestellt) (Anzucht bis zu 80-100%igen konfluenten Zellen) wurden mit Lipofectamin mit den Plasmiden pCIneo, pCIneo_PPP-I und pCIneo_recPrP-I transfiziert. 48 Stunden nach der Transfektion wurden die Zellen geerntet, lysiert und mit 20 ng/µl Proteinase K (Roche Diagnostika) 30 min. bei 37 °C inkubiert.

Die Reaktion wurde mit 1mM Pefabloc abgestoppt und das Protein mit 5 Vol. Methanol 1 h bei -70°C präzipitiert. Das Protein wurde dann durch 20 minütige Zetrifugation bei 14000 rpm (Eppendorf Tischzentrifuge) pelletiert und nachfolgend in 35 µl Bidest resuspendiert. Dann wurden 35 µl SDS-Probenpuffer zugegeben. Nachfolgend wurde das Gesamt-Protein durch Western Blotting unter Entwicklung mit dem 3F4-Antikörper analysiert.

Der Vorteil dieser Zellinie ScN₂a, die das MHM-2 Konstrukt enthält, liegt darin, daß der 3F4-Antikörper der Hamster PrP, aber nicht Maus PrP erkennt, verwendet werden kann, um zwischen dem transgen exprimierten Hamster-PrP und dem endogenen Maus-PrP diskriminieren zu können.

Gleichzeitig mit dem Zeitpunkt, an dem die Zellen, die mit pCIneo (Mock, Spur 1), pCIneo_PPP-I (Spur 2) und pCIneo_recPrP-1 (Spur 3) transfiziert waren, bezüglich der Synthese der PrP^{Sc}-Spiegel analysiert wurden, wurde die zelluläre Gesamt-RNA mit einem peqGOLD RNA PureTM Kit (PeqLab, Deutschland) extrahiert. Nach dieser Präparation von DNA-freier und proteinfreier RNA wurde die RNA mit dem PCR-Primer-I revers transkribiert und anschließend die cDNA mittels der PCR-Primer I und II amplifiziert. 5 Vol-% der erhaltenen DNA wurden auf einem 2,5 % Agarosegel, das 1xTBE und 1µg/ml Ethidiumbromid enthält, analysiert.

### Beispiel 3

### Western- und North-Western-Blot-Analyse

Hirnhomogenate (20%ig in einer Glucoselösung) mit und ohne Behandlung mit 50 µg/ml pK wurden nach Klärung durch Ultraschallbehandlung entweder auf denaturierende SDS-12 % PAA-Gele oder native Polyacrylamidgele aufgetragen. Zur Klärung wurden 20%ige Hirnhomogenate (in isotonischer Glucoselösung) in 2x Verdünnungspuffer (1x Verdünnungspuffer = 1x PBS, 0.5 % DOC, 0,5 % NP40, 200 µM Pefablock und 1 mM PMSF) 1:1 verdünnt. Die Hirnhomogenate wurden anschließend in einem Branson-Beaker-Sonifier mit 40 Pulsen, Stärke 5, Zeitzyklus 30% ultraschallbehandelt. Nach dem Zentrifugieren (7 min mit 15000 min⁻¹ in einer Eppendorf-Tischzentrifuge) wurde der Überstand 1 gewonnen und das Pellet im oben angegebenen 1x Verdünnungspuffer resuspendiert und anschließend mit 40 Pulsen, Stärke 6, Zeitzyklus 40% ultraschallbehandelt. Nach dem oben beschriebenen Zentrifugieren wurden der Überstand 1 und 2 vereinigt, wodurch klares Hirnhomogenat (5 % Glucose) erhalten wurde. Dieses Material wurde auf PAA, TBE-gepufferte Gradientengele gewöhnlich 3.5 bis 12; vorzugsweise 4 bis 12 und insbesondere 6 bis 12 % PAA aufgetragen. Nach Elektroblotten auf Nitrocellulose wurden die Proteinbanden entweder durch Inkubation mit einem radioaktiv markierten RNA-Molekül und anschließende Autoradiographie oder durch Inkubation mit für PrP spezifischen Antikörper (sekundärer Antikörper: jeweils mit POD konjugiertes Anti-Maus-IgG oder Anti-Kaninchen-IgG) und anschließende Chemilumineszenz nachgewiesen.

### Beispiel 4

### Modelle der RNA-Sekundärstruktur

Die Modelle der Sekundärstruktur des PPP-I-Aptamers 73-mer; SEQ ID Nr. 1, 112-mer; SEQ ID Nr. 2 wurde mit dem Software-Programm MacDNASIS Pro V1.0 erstellt (vgl. Figs. 2a + 2b).

Im folgenden Sequenzprotokoll sind die Sequenzen für PPP-I (SEQ ID Nr:1 und 2) und das Kontrollaptamer Ctrl-I (SEQ ID Nr: 3 und 4) angegeben.

### Literaturverzeichnis:

Demainay R. et al., Late Treatment with Polene Antibiotics can prolong the Survival of scrapie-affected Animals. J. Virol. 71 (1997), 9685-9689.

Famulok, M.: Molecular Recognition of Amino Acids by RNA-Aptamers: An L-Citrulline Bindung Motif and its Evolution into an L-Arginine Binder. J. Am. Chem. Soc. 116 (1994), 1698-1706

Gerhart E., Wagner H., Brantl S.:.Kissing and RNA stability in antisense Control of Plasmid Replication. Trends Biochem. Sci. (December 1998), 23 (12): 451-454

Korth C., Stierli B., Streit P., Moser M., Schaller O., Fischer R., Schulz-Schaeffer W., Kretzschmer H., Raeber A., Braun U., Ehrensperger F., Hornemann S., Glockshuber R., Riek R., Billeter M., Wüthrich K., Oesch B.: Prion (PrP^{Sc})-specific epitope defined by a monoclonal antibody. Nature, 390, 74-77 (1997)

King D.J., et al., Novel Combinatorial Selection of Phosphorothioate Oligonucleotide Aptamers. Biochemistry, 37 (1998), 16489-16493.

Kumar M., Carmichael G. G.: Antisense RNA: Function and Fate of Duplex RNA in Cells of higher Eukaryotes. Microbiol. Mol. Bio. 1 Rev. (December 1998), 62 (4): 1415-1434

Lasmezas C. I. et al., Transmission of the BSE Agent to Mice in the Absence of Detectable abnormal Prion Protein. Science 275, 404-405 (1997).

Lasmézas C.I. und Weiss S.: Molecular Biology of Prion Diseases. In: Cary J.W, Linz J.E., Bhatnagar D., eds. Microbial food-borne disease: mechanisms of pathogenesis and toxin synthesis. Lancaster: Technomic Publishing Company, 1999 (in press).

Prusiner, S.B., Scott M.R., DeArmond S.J., and Cohen F.E.: Prion protein biology. Cell, 99, 337-348 (1998).

Rieger R., Lasmezas C.I., Weiss S.: Role of the 37kDa laminin receptor precursor in the life cycle of prions. Transfus. Clin. Biol., 6, 7-16, 1999.

Scott, M.R., Kohler, R., Foster, D., Prusiner, S.B.: Chimeric prion protein expression in cultured cells and transgenic mice. Protein Science, 1, (1992), 986-997.

Tuerk, C. and Gold, L.: Systematic evolution of ligands by exponential enrichment; RNA ligands to bacteriophage T4 DNA polymerase. Science (1990), 249, 505-510.

Vanhee-Brossollet C., Vaquero C.: Do Natural Antisense Transcripts Make Sense in Eukaryotes. Gene (1998), 211 (1):1-9

Weiss S., Proske D., Neumann M., Groschup M., Kretzschmar H., Famulok M. und Winnacker L.: RNA Aptamers Specifically Interact with the Prion Protein PrP. J. Virol. 71 (1997), 8790-8797

Weissmann C., and Aguzzi A.: Bovine spongiform encephalophathy and early onset of varient Creutzfeld-Jakob disease. Curr. Opin. Neurobiol. 7, 695-700 (1997).

### SEQUENZPROTOKOLL

(1) ALLGEMEINE ANGABEN:
   (i) Anmelder:
      (A) Name: Weiss, Stefan
      (B) Strasse: Blütenstr. 20
      (C) Ort: München
      (D) Land: Deutschland
      (E) Postleitzahl: 80799

      (A) Name: Lasmezas, Corinne Ida
      (B) Strasse: 97 Rue de Crimée
      (C) Ort: Paris
      (D) Land: Frankreich
      (E) Postleitzahl: 75019

      Bezeichnung der Erfindung: Nucleinsäuremoleküle mit spezifischer Erkennung von nativem PrP^{Sc}, Herstellung und Verwendung
      Zahl der Sequenzen: 4
      Computerlesbare Fassung:
      (A) Datenträger: Diskette
      (B) Computer: IBM PC kompatibel
      (C) Betriebssystem: PC-DOS/MS-DOS
      (D) Software: WinWord 6.0
(2) INFORMATIONEN FÜR SEQ ID Nr. 1
   (i) SEQUENZEIGENSCHAFTEN:
      (A) Länge: 73 Basenpaare
      (B) Art: Nucleinsäure
      (C) Strangform: Einzelstrang
      (D) Topologie: linear
   (ii) MOLEKÜLTYP: Nucleinsäure
      (A) Beschreibung: RNA
   (iii) Hypothetisch: nein
   (iv) Antisense: nein
   (xi) SEQUENZBESCHREIBUNG: SEQ ID Nr. 1: 
5' GGCAAAGGCGGGAAAGCGUGCUAACGUGGAAAGCUACUCCCACGUUG
UACGCGUCGCAGAUCAUUGAGUGAGG 3'
(2) INFORMATIONEN FÜR SEQ ID Nr. 2
   (i)SEQUENZEIGENSCHAFTEN:
      (A) Länge: 112 Basenpaare
      (B) Art: Nucleinsäure
      (C) Strangform: Einzelstrang
      (D) Topologie: linear
   (ii) MOLEKÜLTYP: Nucleinsäure
      (A) Beschreibung: RNA
   (iii) Hypothetisch: nein
   (iv) Antisense: nein
   (xi) SEQUENZBESCHREIBUNG: SEQ ID Nr:2: 
5' GGAGCUCAGCCUUCACUGCGGCAAAGGCGGGAAAGCGUGCUAACGUG
GAAAGCUACUCCCACGUUGUACGCGUCGCAGAUCAUUGAGUGAGGGGCAC
CACGGUCGGAUCCUC 3'
(2) INFORMATIONEN FÜR SEQ ID NO:3
   (i)SEQUENZEIGENSCHAFTEN:
      (A) Länge: 73 Basenpaare
      (B) Art: Nucleinsäure
      (C) Strangform: Einzelstrang
      (D) Topologie: linear
   (ii) MOLEKÜLTYP: Nucleinsäure
      (A) Beschreibung: RNA
   (iii) Hypothetisch: nein
   (iv) Antisense: nein
   (xi) SEQUENZBESCHREIBUNG: SEQ ID Nr. 3: 
5’ UCCAGGCCGAGUGCAGGAUGUAAUCGAGUCCAUCUGAUUGCUAAUAG
AUUCCUCAUUCAUCAGCACAUAUAGC 3’
(2) INFORMATIONEN FÜR SEQ ID Nr. 4
   (i)SEQUENZEIGENSCHAFTEN:
      (A) Länge: 112 Basenpaare
      (B) Art: Nucleinsäure
      (C) Strangform: Einzelstrang
      (D) Topologie: linear
   (ii) MOLEKÜLTYP: Nucleinsäure
      (A) Beschreibung: RNA
   (iii) Hypothetisch: nein
   (iv) Antisense: nein
   (xi) SEQUENZBESCHREIBUNG: SEQ ID Nr. 4:

5'
GGAGCUCAGCCUUCACUGCGUCCAGGCCGAGUGCAGGAUGUAAUCGAGUC
CAUCUGAUUGCUAAUAGAUUCCUCAUUCAUCAGCACAUAUAGCGGCACCA
CGGUCGGAUCCUC 3'

## Patentansprüche

1. Nucleinsäuremolekül der folgenden Formel:

5' GGCAAAGGCGGGAAAGCGUGCUAACGUGGAAAGCUACUCCCACGUUG
UACGCGUCGCAGAUCAUUGAGUGAGG 3'

2. Nucleinsäuremolekül der folgenden Formel:

5' GGAGCUCAGCCUUCACUGCGGCAAAGGCGGGAAAGCGUGCUAACGUG
GAAAGCUACUCCCACGUUGUACGCGUCGCAGAUCAUUGAGUGAGGGGC
ACCACGGUCGGAUCCUC 3'

3. Nucleinsäuremolekül nach Anspruch 1 oder 2 erhältlich aus pCIneo-PPP-I (hinterlegt unter der Bezeichnung pCIneo-SAF-I, 24. März 1999, DSM 12753).

4. Verwendung eines Nucleinsäuremoleküls nach einem der Ansprüche 1 bis 3 zur Herstellung einer Antisense-RNA.

5. Verwendung der für natives PrP^{Sc} spezifischen Nucleinsäuremoleküle gemäß einem der Ansprüche 1 bis 3 als Mittel zum Nachweis von übertragbaren spongiformen Encephalopathien.

6. Pharmazeutische Zusammensetzung, die Nucleinsäuremoleküle gemäß einem der Ansprüche 1 bis 3 sowie gegebenenfalls pharmazeutisch akzeptable Träger und/oder Hilfsstoffe enthält, als Mittel zur Therapie von übertragbaren spongiformen Encephalopathien bei Tieren und Menschen.

7. Diagnostische Zusammensetzung, die Nucleinsäuremoleküle gemäß einem der Ansprüche 1 bis 3 enthält, zum Nachweis von übertragbaren spongiformen Encephalopathien.

8. Diagnostische Zusammensetzung nach Anspruch 7, wobei der Nachweis in Körperflüssigkeiten erfolgt.

9. Diagnostische Zusammensetzung nach Anspruch 8, wobei es sich bei den Körperflüssigkeiten um Blut, Cerebrospinalflüssigkeit, Milch, Samenflüssigkeit oder Speichel handelt.

10. Verwendung der für natives PrP^{Sc} spezifischen Nucleinsäuremoleküle gemäß einem der Ansprüche 1 bis 3 in einem Verfahren zur Untersuchung der Fähigkeit von Makromolekülen zur selektiven Bindung an PrP^{Sc} unter nativen Bedingungen, wobei das Verfahren die folgenden Stufen umfaßt:
- Ultraschallbehandlung einer Gewebeprobe, um eine klare Lösung der Gewebeprobe herzustellen,
- Trennung der klaren Lösung durch native Gelelektrophorese, um einzelne Proteinbanden zu erhalten, und nachfolgendes Elektroblotten und
- anschließendes Nachweisen von nativem PrP^{Sc}.

11. Verwendung nach Anspruch 10 , wobei die Trennung der klaren Lösung durch Polyacrylamidgelelektrophorese erfolgt.

12. Verwendung nach Anspruch 10, wobei die Trennung der klaren Lösung durch Polyacrylamidgelelektrophorese unter Verwendung eines PAA-Gradienten erfolgt.

13. Verwendung nach Anspruch 10, wobei die Trennung der klaren Lösung durch Agarosegelelektrophorese erfolgt.

14. Verwendung nach Anspruch 10, wobei die Trennung der klaren Lösung durch Agarosegelelektrophorese unter verwendung eines Agarosegradienten erfolgt.

15. Verwendung nach Anspruch 10 bis 14, wobei der Nachweis durch North-Western-Blotting erfolgt.

## Claims

1. Nucleic acid molecule of the following formula:

5′GGCAAAGGCGGGAAAGCGUGCUAACGUGGAAAGCUACUCCCACGUUGU
ACGCGUCGCAGAUCAUUGAGUGAGG 3′

2. Nucleic acid molecule of the following formula:

5′GGAGCUCAGCCUUCACUGCGGCAAAGGCGGAAAGCGUGCUAACGUGG
AAAGCUACUCCCACGUUGUACGCGUCGCAGAUCAUUGAGUGAGGGGCACC
ACGGUCGGAUCCUC 3′

3. Nucleic acid molecule according to Claim 1 or 2 obtainable from pCIneo-PPP-I (deposited under the name pCIneo-SAF-I, 24 March 1999, DSM 12753).

4. Use of a nucleic acid molecule according to any of Claims 1 to 3 for preparing an antisense RNA.

5. Use of the nucleic acid molecules specific for native PrP^{Sc} according to any of Claims 1 to 3 as means for detecting transmissible spongiform encephalopathies.

6. Pharmaceutical composition which comprises nucleic acid molecules according to any of Claims 1 to 3 and, where appropriate, pharmaceutically acceptable carriers and/or excipients as means for the therapy of transmissible spongiform encephalopathies in animals and humans.

7. Diagnostic composition which comprises nucleic acid molecules according to any of Claims 1 to 3 for detecting transmissible spongiform encephalopathies.

8. Diagnostic composition according to Claim 7, where the detection takes place in body fluids.

9. Diagnostic composition according to Claim 8, where the body fluids are blood, cerebrospinal fluid, milk, seminal fluid or saliva.

10. Use of the nucleic acid molecules specific for native PrP^{Sc} according to any of Claims 1 to 3 in a method for investigating the ability of macromolecules to bind selectively to PrP^{Sc} under native conditions, where the method includes the following stages:
- ultrasound treatment of a tissue sample in order to prepare a clear solution of the tissue sample,
- separation of the clear solution by native gel electrophoresis in order to obtain single protein bands, and subsequent electroblotting and
- subsequent detection of native PrP^{Sc}.

11. Use according to Claim 10, where the separation of the clear solution takes place by polyacrylamide gel electrophoresis.

12. Use according to Claim 10, where the separation of the clear solution takes place by polyacrylamide gel electrophoresis with use of a PAA gradient.

13. Use according to Claim 10, where the separation of the clear solution takes place by agarose gel electrophoresis.

14. Use according to Claim 10, where the separation of the clear solution takes place by agarose gel electrophoresis with use of an agarose gradient.

15. Use according to Claim 10 to 14, where the detection takes place by North Western blotting.

## Revendications

1. Molécule d'acide nucléique de formule suivante :

5' GGCAAAGGCGGAAAGCGUGCUAACGUGGAAAGCUACUCCCACGUUG
UACGCGUCGCAGAUCAUUGAGUGAGG 3'

2. Molécule d'acide nucléique de formule suivante :

5' GGAGCUCAGCCUUCACUGCGGCAAAGGCGGAAAGCGUGCUAACGUG
GAAAGCUACUCCCACGUUGUACGCGUCGCAGAUCAUUGAGUGAGGGGCAC
CACGGUCGGAUCCUC 3'

3. Molécule d'acide nucléique selon la revendication 1 ou 2, pouvant être obtenue à partir de pCIneo-PPP-I (déposé sous le nom pCIneo-SAF-I le 24 mars 1999, DSM 12753).

4. Utilisation d'une molécule d'acide nucléique selon l'une quelconque des revendications 1 à 3 pour la préparation d'un ARN antisens.

5. Utilisation des molécules d'acide nucléique spécifiques de la PrP^{Sc} native selon l'une quelconque des revendications 1 à 3 en tant qu'agent pour une détection d'encéphalopathies spongiformes transmissibles.

6. Composition pharmaceutique qui contient des molécules d'acide nucléique selon l'une quelconque des revendications 1 à 3 ainsi qu'éventuellement des véhicules et/ou des supports acceptables sur le plan pharmaceutique, en tant qu'agents pour la thérapie d'encéphalopathies spongiformes transmissibles chez les animaux et chez l'Homme.

7. Composition diagnostique qui contient des molécules d'acide nucléique selon l'une quelconque des revendications 1 à 3 pour une détection d'encéphalopathies spongiformes transmissibles.

8. Composition diagnostique selon la revendication 7, avec laquelle on réalise la détection dans des liquides corporels.

9. Composition diagnostique selon la revendication 8, où les liquides corporels sont le sang, le liquide céphalo-rachidien, le lait, le liquide séminal ou la salive.

10. Utilisation des molécules d'acide nucléique spécifiques de la PrP^{Sc} native selon l'une quelconque des revendications 1 à 3 dans un procédé pour étudier la capacité de macromolécules à se lier de manière sélective à la PrP^{Sc} dans des conditions natives, dans laquelle le procédé comprend les étapes suivantes :
- le traitement aux ultrasons d'un échantillon tissulaire pour préparer une solution transparente de l'échantillon tissulaire,
- la séparation de la solution transparente par électrophorèse native sur gel pour obtenir des bandes protéiques individuelles suivie d'un électrobuvardage, et
- la détection ultérieure de la PrP^{Sc} native.

11. Utilisation selon la revendication 10, dans laquelle on réalise la séparation de la solution transparente par électrophorèse sur gel de polyacrylamide.

12. Utilisation selon la revendication 10, dans laquelle on réalise la séparation de la solution transparente par électrophorèse sur gel de polyacrylamide en utilisant un gradient de PAA.

13. Utilisation selon la revendication 10, dans laquelle on réalise la séparation de la solution transparente par électrophorèse sur gel d'agarose.

14. Utilisation selon la revendication 10, dans laquelle on réalise la séparation de la solution transparente par électrophorèse sur gel d'agarose en utilisant un gradient d'agarose.

15. Utilisation selon les revendications 10 à 14, dans laquelle on réalise la détection par transfert de North-Western.
